# EUROPEAN PATENT SPECIFICATION

(11) **EP 2 060 316 B1**
(45) Date of publication and mention of the grant of the patent: **04.04.2012**
(21) Application number: 08166546.5
(22) Date of filing: 14.10.2008
(51) Int. Cl.: B01D 71/26, B01D 67/00, A61L 2/00, B01D 15/36

(54) **Media for membrane ion exchange chromatography**
Medien für die Membran-Ionaustausch-Chromatografie
Support pour chromatographie d'échange d'ions sur membrane

(30) Priority: 19.11.2007 US 3694 P
(43) Date of publication of application: 20.05.2009
(73) Proprietor: Millipore Corporation, Billerica, MA 01821 (US)
(72) Inventor: Kozlov, Mikhail, Belmont, MA 02478 (US)
(74) Representative: Gambell, Derek

(56) References cited:
- GB-B- 2 390 042
- US-A- 4 431 545
- US-A- 5 137 633
- US-A1- 2003 121 844

## Description

This application claims priority of U.S. Provisional application serial number: 61/003,694 filed November 19, 2007, the disclosure of which is incorporated herein by reference.

### BACKGROUND OF THE INVENTION

Virus purification is an emerging field of bioseparations. Since large amounts of pure viruses are necessary for gene therapy clinical studies, the traditional method of purification, namely, ultracentrifugation, is no longer economical. There is a need to develop faster, less expensive, and more scaleable purification techniques. Chromatography has been used for virus purification, primarily in the format of beads. First reports on chromatography-based virus purification date back about half century (See, for example, Haruna, I.; Yaoi, H.; Kono, R.; Watanabe, I., Separation of adenovirus by chromatography on DEAE-cellulose. *Virology* 1961, 13, (2), 264). Membrane chromatography has started gaining attention recently when capacity and usage limitations of bead chromatography became serious.

Strong anion exchangers, such as those based on quaternary ammonium ions, are used in downstream processing as a polishing media for capturing negatively charged large impurities, such as endotoxins, viruses, nucleic acids, and host cell proteins (HCP) that are present in fluids such as biological fluids, particularly solutions of manufactured biotherapeutics. Traditionally, anion exchangers have been offered and used in the bead format, for example Q Sepharose^{®} available, from GE Healthcare Bio-Sciences AB. However, throughput limitations of bead-based systems require large volume columns to effectively capture impurities.

In bead-based chromatography, most of the available surface area for adsorption is internal to the bead. Consequently, the separation process is inherently slow since the rate of mass transport is typically controlled by pore diffusion. To minimize this diffusional resistance and concomitantly maximize dynamic binding capacity, small diameter beads can be employed. However, the use of small diameter beads comes at the price of increased column pressure drop. Consequently, the optimization of preparative chromatographic separations often involves a compromise between efficiency/dynamic capacity (small beads favored) and column pressure drop (large beads favored).

In contrast, membrane-based chromatographic systems (also called membrane sorbers) have the ligands attached directly to the convective membrane pores, thereby eliminating the effects of internal pore diffusion on mass transport. Additionally, the use of microporous membrane substrates with a tight membrane pore size distribution coupled with effective flow distributors can minimize axial dispersion and provide uniform utilization of all active sites. Consequently, mass transfer rates of membrane sorber media may be an order of magnitude greater than that of standard bead-based chromatography media, allowing for both high efficiency and high-flux separations. Since single or even stacked membranes are very thin compared to columns packed with bead-based media, reduced pressure drops are found along the chromatographic bed, thus allowing increased flow rates and productivities. The necessary binding capacity is reached by using membranes of sufficient internal surface area, yielding device configurations of very large diameter to height ratios (d/h). Since most of the capacity of chromatography beads is internal to the bead, membrane-based chromatography systems gain advantage over beads as the size of adsorbate entities increases (as, for example, in going from a protein molecule to a virus particle).

Properly designed membrane sorbers have chromatographic efficiencies that are 10 - 100 times better than standard preparative bead-based resins. Consequently, to achieve the same level of separation on a membrane sorber, a bed height 10-fold less can be utilized. Bed lengths of 1-5 mm are standard for membrane sorbers, compared to bed heights of 10-30 cm for bead-based systems. Due to the extreme column aspect ratios required for large-volume membrane sorbers, device design is critical. To maintain the inherent performance advantages associated with membrane sorbers, proper inlet and outlet distributors are required to efficiently and effectively utilize the available membrane volume. Membrane sorber technology is ideally suited for this application. Current commercial membrane sorbers, however, suffer from various drawbacks, including low capacity, poor separation from impurities, and difficulty in eluting purified material.

Absorption refers to taking up of matter by permeation into the body of an absorptive material. Adsorption refers to movement of molecules from a bulk phase onto the surface of an adsorptive media. Sorption is a general term that includes both adsorption and absorption. Similarly, a sorptive material or sorption device herein denoted as a sorber, refers to a material or device that either ad- or absorbs or both ad- and absorbs.

A membrane sorber is a highly porous, interconnected media that has the ability to remove (ad- and/or absorb) some components of a solution when the latter flows through its pores. The properties of the membrane sorber and its ability to perform well in the required application depend on the porous structure of the media (skeleton) as well as on the nature of the surface that is exposed to the solution. Typically, the porous media is formed first, from a polymer that does not dissolve or swell in water and possesses acceptable mechanical properties. The porous media is preferably a porous membrane sheet made by phase separation methods well known in the art. See, for example, Zeman LJ, Zydney AL, Microfiltration and Ultrafiltration: Principles and Applications, New York: Marcel Dekker, 1996. Hollow fiber and tubular membranes are also acceptable skeletons. A separate processing step is usually required to modify the external or facial surfaces and the internal pore surfaces of the formed porous structure to impart the necessary adsorptive properties. Since the membrane structure is often formed from a hydrophobic polymer, another purpose of the surface modification step is also to make the surfaces hydrophilic, or water-wettable.

This invention relates to anion exchange chromatography media designed to purify viruses, such as adenoviruses. Adenovirus is a vector of choice in gene therapy studies. It is stable, non-enveloped, and infects cells easily. The most common serotype is labeled Ad5. It is easily expressed in the lab, but requires thorough purification from cell proteins to avoid false positive signals in further transfection studies. Of course, pure adenovirus is also required for its ultimate applications, i.e. gene therapy and vaccination. Electrophoretic studies show that Ad5 is strongly negatively charged at pH around 8, while most species in the cell lysate suspension have weaker charge at this pH. This makes anion exchange chromatography a suitable technique for Ad5 purification.

Anion exchange membranes for virus removal and purification have been prepared previously by chemical grafting technique as taught by US Pat. 7,160,464. It teaches preparation of a membrane engrafted with polymeric side chains having one or more positively charged groups. Those familiar with the art of membrane modification will readily appreciate that a grafting process is specific for every membrane substrate, requires advanced equipment and extensive development work. The present invention offers a significantly simpler approach to creating a positively charged membrane sorber based on direct coating of the membrane. Other prior art teaches preparation of anion exchange membrane without directly linking the charged surface coating to the supporting membrane. US Pat. 6,780,327 teaches preparation of a positively charged membrane comprising a porous substrate and a crosslinked coating including a polymer backbone and pendant positively charged groups, wherein each pendant positively charged group is directly linked to the backbone through a polar spacer group by a single bond. However, the presence of a polar spacer group adds additional modes of interactions between the membrane surface and the sorbent molecule, such as dipole interactions and hydrogen bonding. The latter are very difficult to modulate under the conditions of traditional biological separations. It may be desirable to create a sorptive media that interacts with solution components predominantly by charge interactions, which can be easily modulated and fine-tuned by ionic strength. For example, in a typical application of adenovirus purification, high ionic strength (high salt concentration) is used to elute the virus off the membrane. If other modes of interaction are present, the yield of purified virus may be reduced. Thus, the present invention discloses creating a cross-linked coating on the surface of a microporous membrane that has positively charged groups connected to the backbone of the coating polymer by a single non-polar linker.

We are aware of United States patent US 5137633 (Millipore Corporation) which describes that the surface of a hydrophobic porous substrate is modified with an interpolymeric network of a hydrophilic crosslinked polymer and a crosslinked-polyamine epichlorohydrin resin having fixed positive charges. The hydrophobic substrate is contacted with a reaction system comprising a solution of (a) monomer precursor to the hydrophilic polymer, a nonionic or cationic polymerization initiator and a crosslinking agent and (b) a precursor to the crosslinked positively charged resin. The monomer is polymerized and cross-linked by free radical polymerization followed by heating the contacted substrate to form the charged resin

We are also aware of United States patent publication US 2003/121844 (Saehan Industries Incorporation), which describes a selective membrane having a high fouling resistance. In one embodiment, the selective membrane is a composite polyamide reverse osmosis membrane in which a hydrophilic coating has been applied to the polyamide layer of the membrane, the hydrophilic coating being made by (i) applying to the membrane a quantity of a polyfunctional epoxy compound, the polyfunctional epoxy compound comprising at least two epoxy groups, and (ii) then, cross-linking the polyfunctional epoxy compound in such a manner as to yield a water-insoluble polymer.

### SUMMARY OF THE INVENTION

Essential and optional features of the present invention are set out in the accompanying main- and sub-claims respectively. Thus, the problems of the prior art have been overcome by the present invention, which provides media and devices, such as anion exchangers including such media, wherein the anion exchange coating is formed on a hydrophilic substrate with low non-specific protein binding. The positive charge is connected to the coating backbone by a non-polar linker, and the base membrane material is preferably ultra-high molecular weigh polyethylene. The media operates in a bind-elute mode, with elution being facilitated by high ionic strength. The media provides superior application performance, caustic cleanability, and ease of device manufacturing.

In certain embodiments, the invention relates to porous sorptive media comprising a substrate having a first external side and a second external side, both sides being porous, and a porous thickness between them, the substrate being hydrophilic and having a sorptive material substantially covering the solid matrix of the substrate and the first and second external surfaces, the sorptive material comprising a crosslinked polymer having attached quaternary ammonium functionality through a non-polar linker. In certain embodiments, the cross-linked polymer is modified with a charge-modifying agent comprising an organic compound having quaternary ammonium groups connected by the non-polar linker to a moiety capable of reacting with the cross-linked polymer. The organic compound can have the formula Y-Z-N(CH₃)₃⁺X⁻, wherein Y is a reactive leaving group, Z is a non-polar aliphatic or aromatic linker, and X is a negatively charged ion of a water-soluble acid.

In certain embodiments, the invention relates to a method of purifying a virus, comprising passing a solution comprising the virus through a membrane to adsorb the virus, the membrane comprising a substrate having a first external side and a second external side, both sides being porous, and a porous thickness between them, said substrate being hydrophilic and having a sorptive material substantially covering the solid matrix of the substrate and the first and second external surfaces, the sorptive material comprising a crosslinked polymer having quaternary ammonium functionality through a non-polar linker; washing said membrane with buffer; and eluting said virus off said membrane.

### BRIEF DESCRIPTION OF THE DRAWINGS

Figure 1 is a schematic diagram showing the surface profile of a membrane in accordance with certain embodiments;
Figure 2 is a graph of titration of adenovirus;
Figure 3 is a graph of the amount adsorbed and eluted adenovirus for different virus purification membranes;
Figure 4 is an SDS-PAGE of starting cell lysate, flow-through solution, washing solution and the eluate; and
Figure 5 is a graph of eluted Ad5 as a function of degree of PEI modification with BPTMAB.

### DETAILED DESCRIPTION OF SPECIFIC EMBODIMENTS

The present invention relates to a porous chromatographic or sorptive media having a porous, polymeric coating formed on a porous, self-supporting substrate, and to anionic exchangers including such media. The media is particularly suited for the robust removal of viruses from solutions such as cell lysate.

The porous substrate has two surfaces associated with the geometric or physical structure of the substrate. A sheet will have a top and bottom surface, or a first and a second surface. These are commonly termed "sides." In use, fluid will flow from one side (surface) through the substrate to and through the other side (surface).

The thickness dimension between the two surfaces is porous. This porous region has a surface area associated with the pores. In order to prevent confusion related to the terms "surface", "surfaces", or "surface area," or similar usages, the inventors will refer to the geometric surfaces as external or facial surfaces or as sides. The surface area associated with the pores will be referred to as internal or porous surface area.

Porous material comprises the pores, which are empty space, and the solid matrix or skeleton, which makes up the physical embodiment of the material. For example, in polymer microporous membranes, the phase separated polymer provides the matrix. Herein, the inventors discuss coating or covering the surface of the media. The inventors mean by this that the internal and external surfaces are coated so as to not completely block the pores, that is, to retain a significant proportion of the structure for convective flow. In particular, for the internal surface area, coating or covering means that the matrix is coated or covered, leaving a significant proportion of the pores open.

Absorption refers to taking up of matter by permeation into the body of an absorptive material. Adsorption refers to movement of molecules from a bulk phase onto the surface of an adsorptive media. Sorption is a general term that includes both adsorption and absorption. Similarly, a sorptive material or sorption device herein denoted as a sorber, refers to a material or device that both ad- and absorbs.

The membrane chromatography media of the present invention includes an anion exchange coating formed on a porous substrate. The porous substrate acts as a supporting skeleton for the coating. The substrate should be amenable to handling and manufacturing into a robust and integral device. The pore structure should provide for uniform flow distribution, high flux, and high surface area. The substrate is preferably a sheet formed of a membrane. The preferred substrate is made from synthetic or natural polymeric materials. Thermoplastics are a useful class of polymers for this use. Thermoplastics include but are not limited to polyolefins such as polyethylenes, including ultrahigh molecular weight polyethylenes, polypropylenes, sheathed polyethylene/polypropylene fibers, PVDF, polysulfone, polyethersulfones, polyarylsulphones, polyphenylsulfones, polyvinyl chlorides, polyesters such as polyethylene terephthalate, polybutylene terephthalate and the like, polyamides, acrylates such as polymethylmethacrylate, styrenic polymers and mixtures of the above. Other synthetic materials include celluloses, epoxies, urethanes and the like. The substrate also should have low non-specific protein binding.

Suitable substrates include microporous filtration membranes, i.e. those with pore sizes from about 0.1 to about 10 µm. Substrate material can be hydrophilic or hydrophobic. Examples of hydrophilic substrate materials include, but are not limited to, polysaccharides and polyamides, as well as surface treated hydrophilic porous membranes, such as Durapore® (Millipore Corporation, Billerica MA). Examples of hydrophobic material include, but are not limited to, polyolefins, polyvinylidene fluoride, polytetafluoroethylene, polysulfones, polycarbonates, polyesters, polyacrylates, and polymethacrylates. The porous structure is created from the substrate material by any method known to those skilled in the art, such as solution phase inversion, temperature-induced phase separation, air casting, track-etching, stretching, sintering, laser drilling, etc. Because of the universal nature of the present invention, virtually any available method to create a porous structure is suitable for making the supporting skeleton for the membrane sorber. A substrate material made from ultra-high molecular weight polyethylene has been found to be particularly useful due to its combination of mechanical properties, chemical, caustic and gamma stability. Where hydrophobic substrates are used, they should be rendered hydrophilic, such as by a modification process known to those skilled in the art. Suitable modification processes are disclosed in U.S. Patent Nos. 4,618,533 and 4,944,879. A low-protein binding surface hyddrophilization of the substrate (e.g., <50 µg/cm² protein binding) is preferred.

The coating polymer forms the adsorptive hydrogel and bears the chemical groups (binding groups) responsible for attracting and holding the impurities. Alternatively, the coating polymer possesses chemical groups that are easily modifiable to incorporate the binding groups. The coating is permeable to biomolecules so that proteins and other impurities can be captured into the depth of the coating, increasing adsorptive capacity. The preferred coating polymer is branched or unbranched polyethylene imine.

The coating typically constitutes at least about 3% of the total volume of the coated substrate, preferably from about 5% to about 10%, of the total volume of the substrate. In certain embodiments, the coating covers the substrate in a substantially uniform thickness. Suitable thicknesses range of dry coating from about 10 nm to about 50 nm.

A cross-linker reacts with the polymer to make the latter insoluble in water and thus held on the surface of the supporting skeleton. Suitable crosslinkers include those with low protein binding properties, such as polyethylene glycol diglycidyl ether (PEG-DGE). The amount of cross-linker used in the coating solution is based on the molar ratio of reactive groups on the polymer and on the cross-linker. The preferred ratio is in the range from about 20 to about 2000, more preferred from about 40 to about 400, most preferred from about 80 to about 200. More cross-linker will hinder the ability of the hydrogel to swell and will thus reduce the sorptive capacity, while less cross-linker may result in incomplete cross-linking, i.e. leave some polymer molecules fully soluble.

The immobilized coating is then modified with a charge-modifying agent in order to impart quaternary ammonium functionality to the coating for suitable membrane chromatography applications. Suitable charge-modifying agents are organic compounds with quaternary ammonium groups connected by a non-polar linker to another moiety capable of reacting with the immobilized coating. These compounds have a general formula Y-Z-N(Alk)₃⁺X⁻ where Y is a reactive leaving group, Z is a non-polar aliphatic or aromatic linker, and X is an anion of any water-soluble acid. The purpose of the leaving group Y is to facilitate reaction between the ligand and the membrane coating and then depart causing the formation of a direct bond between the linker and the coating. A "good" leaving group is usually one that favors high reaction yield under relatively mild conditions. Examples of leaving groups Y include halogens such as Br-, Cl-, I-, F-, and sulfonyl derivatives (TsO-, CF₃SO₃-, C₄F₉SO₃- etc.). The chemistry of leaving groups is well studied; see, for example, M.B. Smith and J. March, Comprehensive Organic Chemistry, 5th ed., Wiley Interscience, 2001. A catalyst is normally required to effect the coupling reaction and promote departure of the leaving group. Acids or bases can serve as catalysts depending on the nature of the reaction. When the starting coating constitutes a polymeric amine, a basic catalyst is usually needed to enhance the nucleophilic character of the amine nitrogen. This basic catalyst can be any strong inorganic base (hydroxides of lithium, sodium, potassium, calcium, barium) or organic base (tetra-alkyl ammonium hydroxide). The non-polar linker can be any saturated or unsaturated aliphatic hydrocarbon, for example (CH₂)ₙ where n is from 2 to 10, a branched aliphatic hydrocarbon such as - (CH₂)ₙ-C(CH₃)₂-, an aromatic group such as phenylene, tolylene, xylylene, or a combination of an aliphatic and aromatic. The quaternary ammonium group -N(Alk)₃+ is preferably a trimethyl ammonium group, but can also include other alkyl or aryl groups such as ethyl, phenyl, benzyl, hydroxyethyl, etc. Anion X is an anion of any water-soluble organic or inorganic acid. Examples of suitable anions X include, but are not limited to, chloride, bromide, iodide, acetate, propionate, hydrogen phosphate, hydrogen sulfate, citrate, bicarbonate, methyl sulfonate, sulfamate, etc. Examples of suitable charge-modifying compounds include 2-chloroethyltrimethyl ammonium chloride (chlorocholine chloride), 2-bromoethyltrimethyl ammonium chloride, 3-chloropropyltrimethylammonium chloride (CPTMAC), and 3-bromopropyltrimethylammonium bromide (BPTMAB) A preferred charge-modifying agent is 3-bromopropyltrimethyl ammonium bromide (BPTMAB).

The degree of modification, i.e. the percentage of reactive groups on the cross-linked coating that react with the charge-modifying compound, has to be high enough to ensure that the solute primarily interacts with the membrane surface by charge interactions. For example, PEI has hydrogen-bonding donor groups (secondary amines) which may reduce the yield of eluted virus if they are not converted into and/or covered by quaternary ammonium groups. A preferred degree of modification is at least 10%, more preferred at least 20%, and most preferred at least 30%. Due to the relative sizes of a PEI repeat unit and BPTMAB (steric constraints), it is virtually impossible to achieve a degree of modification much higher than 50%.

A preferred process for forming the coated substrate comprises the steps of: 1) Preparing a solution of the coating polymer and a cross-linker, and adjusting the pH so that polymer readily reacts with cross-linker; 2) Submerging the porous structure into the solution from 1); 3) Removing the porous structure from solution and nipping off the excess liquid; 4) Drying the porous structure to effect cross-linking; 5) Submerging the porous structure in solution containing the charge-modifying compound for a specified period of time; 6) Removing the porous structure from the solution of charge-modifying compound, rinsing with water and drying.

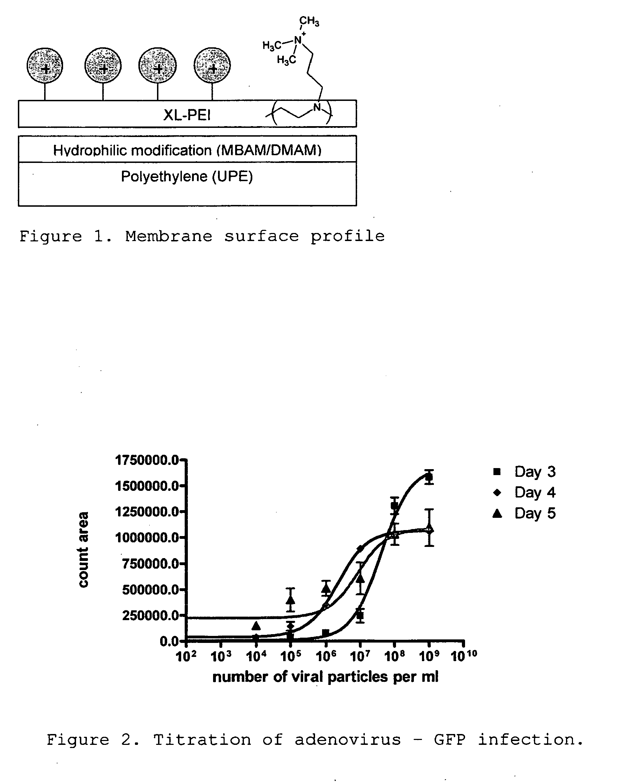

Turning now to Figure 1, the structure of a membrane in accordance with certain embodiments is illustrated. In the embodiment shown, a microporous ultrahigh molecular weight polyethylene membrane was first modified by copolymerizing dimethylacrylamide and methylene-bis-acrylamide on its surface using a free radical initiator and UV activation. Such membranes modified in this manner have a pore size rating of 0.65 µm and are commercially available from Entegris, Inc., and are designated MPLC. Such membranes are characterized by low protein binding to its surface; IgG binding to this membrane is 40-50 µg/cm², which is approximately 2-3 times higher than DURAPORE^{®} membranes, but 6-7 times lower than Immobilon P and other similarly hydrophobic, high-binding membranes that are commercially available.

The modified membrane was coated with a solution containing polyethyleneimine (PEI) and a cross-linker, polyethylene glycol diglycidyl ether (PEG-DGE). The coating was dried and cured at room temperature for 24 hours, rinsed with water, and further modified with 3-bromopropyltrimethylammonium bromide (BPTAB) in 50% aqueous solution at pH 13 maintained with sodium hydroxide.

The resulting membrane has a high density of positive charge on the surface as indicated by high adsorption of negative dyes, for example Ponceau S. The membrane is stable in caustic media and could be fabricated in a wide range of devices. It can be easily pleated, heat-sealed or overmolded.

The following examples are included herein for the purpose of illustration and are not intended to limit the invention.
Example 1. A 6x6" sheet of hydrophilized polyethylene membrane with pore size rating 0.65 um was coated with aqueous solution containing 7 wt.% of polyethyleneimine (Sigma-Aldrich), 0.35% of polyethylene glycol diglycidyl ether (Sigma-Aldrich), and 0.03M of sodium hydroxide. Excess of solution was nipped off and the membrane was allowed to dry overnight. It is subsequently rinsed with water and submerged in 100 mL of 50 wt% solution of 3-bromopropyltrimethylammonium bromide (BPTMAB) and 0.1M sodium hydroxide. The membrane was left in this solution for 48 hrs, and concentrated NaOH was periodically added to maintain pH at 13. The membrane was then removed from solution, rinsed with water, and dried.
Example 2. Membrane prepared in Example 1 was used for adenovirus purification. Adenovirus was first extracted from the infected cells by multiple cycles of freezing and thawing. The cellular debris was removed by centrifugation leaving the viable virus particles in the supernatant. Supernatant was treated with Benzonase. The supernatant was further clarified by passing it through a microporous 0.2 um membrane filter. The solution was diluted with the equilibration buffer, pH 8.0, NaCl concentration 100mM. The same buffer was used for conditioning the purification membrane. Virus solution was slowly passed through the membrane that adsorbs the virus particles, allowing much of the cellular debris to pass through the filter. The membrane was then washed with a wash buffer, pH 8.0, NaCl concentration 200-250mM, to remove any weakly bound debris. Finally, the virus was eluted off the membrane with an elution buffer. pH 8.0, NaCl concentration 1000 mM.

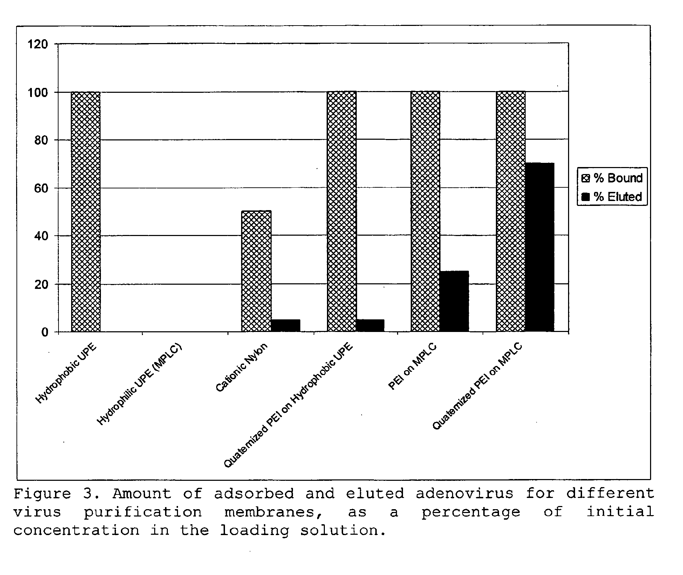

Virus concentration was assessed by Green Fluorescent Protein (GFP) assay, which was developed in house. Figure 2 shows how the area of green fluorescence (observed under microscope) correlates with the concentration of virus particles. The majority of the data was obtained with 3-day GFP assay. Virus retention and elution data is presented in Figure 3.

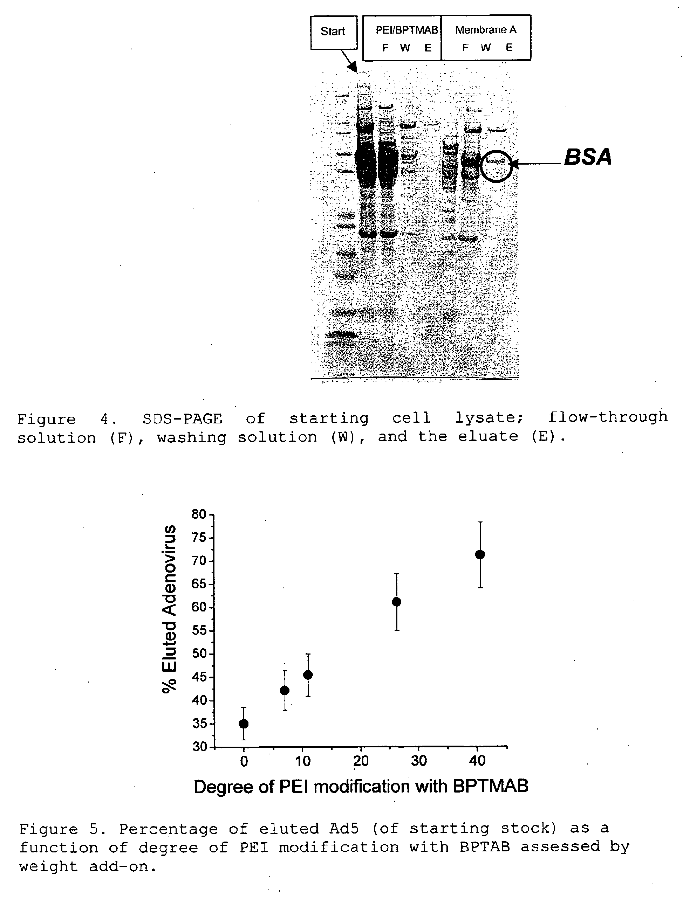

One of the features of the sorptive media of the present invention is the high yield and purity of produced adenovirus. Open bars in Fig. 3 correspond to captured adenovirus from the cell lysate while the solid bars indicate the percentage of virus recovered from the membrane. High virus recovery (> 70%) indicated by this data makes this media very suitable for adenovirus application.
Purity of virus particles was analyzed by gel electrophoresis, which is shown in Fig. 4. It is seen that the membrane of the present invention, PEI-BPTMAB, provides high purity of eluted virus suspension, which is superior to a commercial membrane A as indicated by a less pronounced BSA band.
Example 4. Membranes were prepared according to Example 1 using variable concentration of BPTMAB in the reaction mixture, which produced different degrees of modification. Figure 5 shows that the degree of PEI modification with BPTMAB has a direct impact on the percentage of eluted virus.

## Claims

1. A porous sorptive media comprising a substrate having a first external side and a second external side, both sides being porous, and a porous thickness between them, said substrate being hydrophilic and having a sorptive material substantially covering the solid matrix of the substrate and said first and second external surfaces, said sorptive material comprising a crosslinked polymer having quaternary ammonium groups attached through a non-polar linker.

2. The porous sorptive media of Claim 1, wherein said substrate comprises a microporous membrane.

3. The porous sorptive media of Claim 2, wherein said membrane comprises a polyolefin.

4. The porous sorptive media of Claim 3, wherein said polyolefin is polyethylene.

5. The porous sorptive media of claim 1, wherein said substrate is an ultrahigh molecular weight polyethylene membrane.

6. The porous coated media of any preceding claim, wherein said crosslinked polymer comprises polyethyleneimine.

7. The porous coated media of any preceding claim, wherein said cross-linked polymer is modified with a charge-modifying agent comprising an organic compound having quaternary ammonium groups connected by said non-polar linker to a moiety capable of reacting with said cross-linked polymer.

8. The porous coated media of claim 7, wherein said organic compound has the formula Y-Z-N (CH₃)₃⁺X⁻, wherein Y is a reactive leaving group, Z is a non-polar aliphatic or aromatic linker, and X is a negatively charged ion of a water-soluble acid.

9. The porous coated media of claim 8, wherein Y is a member selected from the group consisting of Br-, Cl-, I-, TsO-and CF₃SO₃-, and Z is (CH₂)ₙ where *n* is from 2 to 10.

10. The porous coated media of claim 6, wherein the quaternary ammonium groups are formed on said polyethyleneimine by 3-bromopropyltrimethyl ammonium bromide.

11. A method of purifying a virus, comprising passing a solution comprising said virus through a membrane to adsorb said virus, said membrane comprising a substrate having a first external side and a second external side, both sides being porous, and a porous thickness between them, said substrate being hydrophilic and having a sorptive material substantially covering the solid matrix of the substrate and said first and second external surfaces, said sorptive material comprising a crosslinked polymer having quaternary ammonium groups attached through a non-polar linker; washing said membrane with buffer; and eluting said virus off said membrane.

12. The method of claim 11, wherein said cross-linked polymer is modified with a charge-modifying agent comprising an organic compound having quaternary ammonium groups connected by said non-polar linker to a moiety capable of reacting with said cross-linked polymer.

13. The method of claim 12, wherein said organic compound has the formula Y-Z-N (CH₃)₃⁺X⁻, wherein Y is a reactive leaving group, Z is a non-polar aliphatic or aromatic linker, and X is a negatively charged ion of a monovalent water-soluble acid.

14. An anion exchanger comprising porous sorptive media comprising a substrate having a first external side and a second external side, both sides being porous, and a porous thickness between them, said substrate being hydrophilic and having a sorptive material substantially covering the solid matrix of the substrate and said first and second external surfaces, said sorptive material comprising a crosslinked polymer having quaternary ammonium groups attached through a non-polar linker.

## Patentansprüche

1. Poröses Sorptionsmedium, das ein Substrat mit einer ersten Außenseite und einer zweiten Außenseite, wobei beide Seiten porös sind, und einer porösen dicken Lage zwischen diesen umfasst, wobei das Substrat hydrophil ist und ein Sorptionsmaterial aufweist, das die feste Matrix des Substrats und die erste und die zweite äußere Oberfläche im Wesentlichen bedeckt, wobei das Sorptionsmaterial ein vernetztes Polymer mit über einen nichtpolaren Linker angebrachten quaternären Ammoniumgruppen umfasst.

2. Poröses Sorptionsmedium nach Anspruch 1, wobei das Substrat eine mikroporöse Membran umfasst.

3. Poröses Sorptionsmedium nach Anspruch 2, wobei die Membran ein Polyolefin umfasst.

4. Poröses Sorptionsmedium nach Anspruch 3, wobei das Polyolefin Polyethylen ist.

5. Poröses Sorptionsmedium nach Anspruch 1, wobei das Substrat eine Membran aus Polyethylen von ultrahohem Molekulargewicht ist.

6. Poröses beschichtetes Medium nach einem der vorhergehenden Ansprüche, wobei das vernetzte Polymer Polyethylenimin umfasst.

7. Poröses beschichtetes Medium nach einem der vorhergehenden Ansprüche, wobei das vernetzte Polymer mit einem Ladungsmodifizierungsmittel modifiziert ist, das eine organische Verbindung mit quaternären Ammoniumgruppen, die durch den nichtpolaren Linker mit einer Einheit verbunden sind, die mit dem vernetzten Polymer reagieren kann, umfasst.

8. Poröses beschichtetes Medium nach Anspruch 7, wobei die organische Verbindung die Formel Y-Z-N(CH₃)₃⁺X⁻ aufweist, worin Y für eine reaktive Abgangsgruppe steht, Z für einen nichtpolaren aliphatischen oder aromatischen Linker steht und X für ein negativ geladenes Ion einer wasserlöslichen Säure steht.

9. Poröses beschichtetes Medium nach Anspruch 8, wobei Y für eine Gruppe steht, die aus Br⁻, Cl⁻, I⁻, TsO⁻ und CF₃SO₃⁻ ausgewählt ist, und Z für (CH₂)ₙ steht, wobei n für 2 bis 10 steht.

10. Poröses beschichtetes Medium nach Anspruch 6, wobei die quaternären Ammoniumgruppen an dem Polyethylenimin durch 3-Brompropyltrimethylammoniumbromid ausgebildet werden.

11. Verfahren zur Reinigung eines Virus, umfassend das Hindurchführen einer Lösung, die das Virus umfasst, durch eine Membran zur Adsorption des Virus, wobei die Membran ein Substrat mit einer ersten Außenseite und einer zweiten Außenseite, wobei beide Seiten porös sind, und einer porösen dicken Lage zwischen diesen umfasst, wobei das Substrat hydrophil ist und ein Sorptionsmaterial aufweist, das die feste Matrix des Substrats und die erste und die zweite äußere Oberfläche im Wesentlichen bedeckt, wobei das Sorptionsmaterial ein vernetztes Polymer mit über einen nichtpolaren Linker angebrachten quaternären Ammoniumgruppen umfasst; das Waschen der Membran mit einem Puffer und das Eluieren des Virus von der Membran.

12. Verfahren nach Anspruch 11, wobei das vernetzte Polymer mit einem Ladungsmodifizierungsmittel modifiziert ist, das eine organische Verbindung mit quaternären Ammoniumgruppen, die durch den nichtpolaren Linker mit einer Einheit verbunden sind, die mit dem vernetzten Polymer reagieren kann, umfasst.

13. Verfahren nach Anspruch 12, wobei die organische Verbindung die Formel Y-Z-N(CH₃)₃⁺X⁻ aufweist, worin Y für eine reaktive Abgangsgruppe steht, Z für einen nichtpolaren aliphatischen oder aromatischen Linker steht und X für ein negativ geladenes Ion einer einwertigen wasserlöslichen Säure steht.

14. Anionenaustauscher, umfassend ein poröses Sorptionsmedium, das ein Substrat mit einer ersten Außenseite und einer zweiten Außenseite, wobei beide Seiten porös sind, und einer porösen dicken Lage zwischen diesen umfasst, wobei das Substrat hydrophil ist und ein Sorptionsmaterial aufweist, das die feste Matrix des Substrats und die erste und die zweite äußere Oberfläche im Wesentlichen bedeckt, wobei das Sorptionsmaterial ein vernetztes Polymer mit über einen nichtpolaren Linker angebrachten quaternären Ammoniumgruppen umfasst.

## Revendications

1. Support sorbant poreux comprenant un substrat qui présente un premier côté externe et un second côté externe, les deux côtés étant poreux, et une épaisseur poreuse entre eux, ledit substrat étant hydrophile et ayant un matériau sorbant qui couvre sensiblement la matrice solide du substrat et lesdites première et seconde surfaces externes, ledit matériau sorbant comprenant un polymère réticulé comportant des groupes d'ammonium quaternaire fixés par un agent de liaison non polaire.

2. Support sorbant poreux selon la revendication 1, dans lequel ledit substrat comprend une membrane microporeuse.

3. Support sorbant poreux selon la revendication 2, dans lequel ladite membrane comprend une polyoléfine.

4. Support sorbant poreux selon la revendication 3, dans lequel ladite polyoléfine est le polyéthylène.

5. Support sorbant poreux selon la revendication 1, dans lequel ledit substrat est une membrane en polyéthylène de masse moléculaire très élevée.

6. Support poreux revêtu selon l'une quelconque des revendications précédentes, dans lequel ledit polymère réticulé comprend une polyéthylèneimine.

7. Support poreux revêtu selon l'une quelconque des revendications précédentes, dans lequel ledit polymère réticulé est modifié par un agent de modification de charge comprenant un composé organique comportant des groupes d'ammonium quaternaire reliés par ledit agent de liaison non polaire à une fraction apte à réagir avec ledit polymère réticulé.

8. Support poreux revêtu selon la revendication 7, dans lequel ledit composé organique a la formule Y-Z-N(CH₃)₃⁺X⁻, dans laquelle Y est un groupe partant réactif, Z est un agent de liaison aliphatique ou aromatique non polaire, et X est un ion de charge négative d'un acide hydrosoluble.

9. Support poreux revêtu selon la revendication 8, dans lequel Y est un élément choisi dans le groupe constitué de Br-, Cl-, I-, TsO- et CF₃SO₃-, et Z est (CH₂)ₙ où n va de 2 à 10.

10. Support poreux revêtu selon la revendication 6, dans lequel les groupes d'ammonium quaternaire sont formés sur ladite polyéthylèneimine par du bromure d'ammonium 3-bromopropyltriméthylique.

11. Procédé de purification d'un virus, comprenant l'étape qui consiste à faire passer une solution comprenant ledit virus à travers une membrane pour adsorber ledit virus, ladite membrane comprenant un substrat qui présente un premier côté externe et un second côté externe, les deux côtés étant poreux, et une épaisseur poreuse entre eux, ledit substrat étant hydrophile et ayant un matériau sorbant qui couvre sensiblement la matrice solide du substrat et lesdites première et seconde surfaces externes, ledit matériau sorbant comprenant un polymère réticulé comportant des groupes d'ammonium quaternaire fixés par un agent de liaison non polaire ; à laver ladite membrane avec un tampon ; et à éluer ledit virus de ladite membrane.

12. Procédé selon la revendication 11, dans lequel ledit polymère réticulé est modifié par un agent de modification de charge comprenant un composé organique comportant des groupes d'ammonium quaternaire reliés par ledit agent de liaison non polaire à une fraction apte à réagir avec ledit polymère réticulé.

13. Procédé selon la revendication 12, dans lequel ledit composé organique a la formule Y-Z-N(CH₃)₃⁺X⁻, dans laquelle Y est un groupe partant réactif, Z est un agent de liaison aliphatique ou aromatique non polaire, et X est un ion de charge négative d'un acide hydrosoluble monovalent.

14. Echangeur d'anions comprenant un support sorbant poreux comprenant un substrat qui présente un premier côté externe et un second côté externe, les deux côtés étant poreux, et une épaisseur poreuse entre eux, ledit substrat étant hydrophile et ayant un matériau sorbant qui couvre sensiblement la matrice solide du substrat et lesdites première et seconde surfaces externes, ledit matériau sorbant comprenant un polymère réticulé comportant des groupes d'ammonium quaternaire fixés par un agent de liaison non polaire.
